(19) Europäisches Patentamt
European Patent Office
Office européen des brevets

(11) **EP 2 829 533 A1**

(12) **EUROPÄISCHE PATENTANMELDUNG**

(43) Veröffentlichungstag:
**28.01.2015 Patentblatt 2015/05**

(51) Int Cl.:
***C07C 263/10*** *(2006.01)*

(21) Anmeldenummer: **13178270.8**

(22) Anmeldetag: **26.07.2013**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Bayer MaterialScience AG
51373 Leverkusen (DE)**

(72) Erfinder: **Die Erfindernennung liegt noch nicht vor**

(74) Vertreter: **BIP Patents
c/o Bayer Intellectual Property GmbH
Creative Campus Monheim
Alfred-Nobel-Straße 10
40789 Monheim (DE)**

(54) **Verfahren zur herstellung von isocyanaten**

(57) Gegenstand der Erfindung ist ein Verfahren zur Herstellung eines Isocyanats durch Umsetzung des korrespondierenden primären Amins mit Phosgen in einem Reaktor **100**, der mindestens eine Reaktionszone **110** und eine darunter angeordnete Quenchzone **120** umfasst, umfassend die Schritte:

(i) Einleiten eines gasförmigen Aminstroms **1** und eines gasförmigen Phosgenstroms **2** in den Reaktor **100** und Umsetzung in der Reaktionszone **110** zu einem Produktgasstrom **3** umfassend Isocyanat und Chlorwasserstoff sowie ggf. überschüssiges Phosgen;

(ii) Einleiten des Produktgasstromes **3** in die Quenchzone **120**, in welcher der Produktgasstrom durch Eindüsen einer Quenchflüssigkeit **4** über mindestens eine Quenchdüse **200** so abgekühlt wird, dass ein flüssiger Strom **5** umfassend Quenchflüssigkeit **4** und Isocyanat sowie ein gasförmiger Strom **6** umfassend Chlorwasserstoff und ggf. Phosgen erhalten werden;

(iii) Isolierung des Isocyanats aus dem in Schritt (ii) erhaltenen flüssigen Strom **5**;

bei dem die Temperatur der Wand der Reaktionszone **110** unmittelbar oberhalb der Quenchzone **120**, $T_w{}^*$, auf einem Wert gehalten wird, der maximal 4,0 %, bevorzugt maximal 2,0 %, unter der in Kelvin angegebenen Maximaltemperatur der Wand der Reaktionszone, $T_w{}^{max}$, liegt.

EP 2 829 533 A1

**Beschreibung**

Gegenstand der Erfindung ist ein Verfahren zur Herstellung eines Isocyanats durch Umsetzung des korrespondierenden primären Amins mit Phosgen in einem Reaktor **100,** der mindestens eine Reaktionszone **110** und eine darunter angeordnete Quenchzone **120** umfasst, umfassend die Schritte:

(i) Einleiten eines gasförmigen Aminstroms **1** und eines gasförmigen Phosgenstroms **2** in den Reaktor **100** und Umsetzung in der Reaktionszone **110** zu einem Produktgasstrom **3** umfassend Isocyanat und Chlorwasserstoff sowie ggf. überschüssiges Phosgen;

(ii) Einleiten des Produktgasstromes **3** in die Quenchzone **120,** in welcher der Produktgasstrom durch Eindüsen einer Quenchflüssigkeit **4** über mindestens eine Quenchdüse **200** so abgekühlt wird, dass ein flüssiger Strom **5** umfassend Quenchflüssigkeit **4** und Isocyanat sowie ein gasförmiger Strom 6 umfassend Chlorwasserstoff und ggf. Phosgen erhalten werden;

(iii) Isolierung des Isocyanats aus dem in Schritt (ii) erhaltenen flüssigen Strom **5;**

bei dem die Temperatur der Wand der Reaktionszone **110** unmittelbar oberhalb der Quenchzone **120, $T_W^*$,** auf einem Wert gehalten wird, der maximal 4,0 %, bevorzugt maximal 2,0 %, unter der in Kelvin angegebenen Maximaltemperatur der Wand der Reaktionszone, $T_W^{max}$, liegt.

Die Herstellung von Diisocyanaten durch Umsetzung von Diaminen in der Gasphase wird beispielsweise in EP 0289840 B1 beschrieben. Die in einem Rohrreaktor gebildeten Diisocyanate sind bei den Reaktionstemperaturen von bis zu 500 °C thermisch nicht stabil. Eine rasche Abkühlung der Reaktionsgase nach der Phosgenierungsreaktion auf Temperaturen unter 150 °C ist daher notwendig, um die Bildung unerwünschter Nebenprodukte durch den thermischen Zerfall von Diisocyanat oder durch eine Weiterreaktion zu vermeiden. In EP 0289840 B1 oder EP 0749 958 B1 wird dazu das den Reaktionsraum kontinuierlich verlassende gasförmige Gemisch, welches u. a. Diisocyanat, Phosgen und Chlorwasserstoff enthält, in ein inertes Lösungsmittel, z. B. Dichlorbenzol, eingeleitet. Nachteilig bei diesem Verfahren ist, dass die Strömungsgeschwindigkeit, mit der das Gasgemisch durch das Lösungsmittelbad geleitet wird, relativ niedrig gewählt werden muss, da bei zu hohen Geschwindigkeiten Lösungsmittel und die darin gelösten Verbindungen mitgerissen würden. In einem nachfolgenden Schritt müssten die flüssigen Verbindungen vom Gas abgetrennt werden. Ein weiterer Nachteil ist, dass aufgrund der niedrigen Strömungsgeschwindigkeit und eines geringen Wärmeübergangsterms große Lösungsmittelbehälter eingesetzt werden müssen, um die Abkühlung zu erzielen.

Weiterhin sind Verfahren bekannt, die zur Abkühlung der Reaktionsgase Wärmeaustauscher einsetzen und/oder die Gase im Vakuum entspannen (DE 10158160 A1). Der Nachteil von Wärmeaustauschern liegt darin, dass wegen des schlechten Wärmeübergangs große Austauschflächen, und damit große Wärmetauscher, für eine effektive Kühlung benötigt werden. Außerdem kann es zu Ablagerungen von Feststoffen auf den verhältnismäßig kalten Flächen der Wärmetauscher durch Nebenreaktionen des Gasgemisches, wie z. B. Zersetzung, Polymerisation oder Ausfällung, kommen.

EP 1 761 483 B1 versucht, die Verweilzeit zwischen Reaktionsende und Abkühlzone dadurch zu verringern, dass sich zwischen der Reaktionszone und der Zone, in der der Reaktionsabbruch herbeigeführt wird, ein Bereich mit reduziertem Strömungsquerschnitt befindet.

Die in der Anmeldung WO2007/014936 A2 beschriebene Reaktionsabbruchzone (sog. "Quenche") ist ein Bereich, in dem die heißen Produktgase durch Einsprühen einer Quenchflüssigkeit rasch abgekühlt werden. Als mögliche Quench-Flüssigkeiten werden Lösungsmittel, Isocyanat-Gemische und Lösungsmittel-Isocyanat-Gemische genannt. Erwähnt wird das Eindüsen einer Quench-Flüssigkeit zum Abkühlen des Reaktionsgemisches und selektiven Lösen des gebildeten Diisocyanats im Lösungsmittel, wobei eine erste Trennung in eine Flüssigphase und eine Gasphase mit überwiegend Phosgen und Chlorwasserstoff als Bestandteilen erfolgt. Die beiden Phasen werden danach einer entsprechenden Aufarbeitung zugeführt. Auf Optimierungsmöglichkeiten dieses Verfahrensschrittes wird nicht eingegangen.

Ein weiterentwickeltes Verfahren zum raschen Abkühlen des gasförmigen Reaktionsgemisches bietet das Eindüsen einer oder mehrerer Quench-Flüssigkeiten in das kontinuierlich aus der Reaktionszone in die nachgeschaltete Quench-Zone strömende Gasgemisch, wie in WO 2011/003532 A1 erwähnt und in EP 1403 248 B1 näher beschrieben ist.

Das Eindüsen von Quench-Flüssigkeit mit Hilfe von mindestens zwei Sprühdüsen, welche am Eingang der Quench-Zone angeordnet sind, wird in EP 1 403 248 B1 offenbart. Hierbei eignen sich als Quench-Flüssigkeiten organische Lösungsmittel oder eine Mischung verschiedener organischer Lösungsmittel, welche mit dem gebildeten Diisocyanat nicht reagieren, wie in EP 1 403 248 B1 beschrieben. Eine Lösung des gebildeten Diisocyanats in einem geeigneten organischen Lösungsmittel kann auch verwendet werden, was die Menge von eingesetztem Lösungsmittel reduziert. Der Durchmesser der Quench-Zone kann größer oder kleiner sein als der Durchmesser der Reaktionszone. Das Quenchen der Reaktionsgase kann einstufig und auch mehrstufig erfolgen. In dieser Schrift wird darauf verwiesen, dass das Einsprühverfahren der Quenchflüssigkeit in das heiße Reaktionsgas derart erfolgt, dass ein Kontakt des Reaktionsgases mit der kalten Wandung vermieden wird. Dadurch wird das Entstehen von Ablagerungen vermieden.

Optimiert wird dieses System in EP 1 935 875 B1 dadurch, dass zum Abbruch der Reaktion das Reaktionsgemisch aus dem Reaktionsraum heraus durch eine Kühlstrecke geführt wird, in die hinein Flüssigkeiten eingedüst werden, sodass die direkte Kühlung in der Kühlstrecke einstufig (d. h. nur ein Kondensationsgemisch erhaltend) in zwei oder mehreren hintereinander geschalteten Kühlzonen erfolgt. Das erzeugte Diisocyanat wird dabei in einem gemeinsamen Kondensationsgemisch erhalten. Dieses Gemisch wird vorzugsweise in einem Flüssigkeitssammelbehälter, angeordnet unterhalb der Kühlstrecke, aufgefangen. Dieses Kondensationsgemisch kann zur Abtrennung des hergestellten Isocyanats ausgeschleust oder, bevorzugt nach erfolgter Kühlung, teilweise auf eine oder mehrere Kühlzonen der Kühlstrecke zurückgeführt werden. Nachteilig an diesem Einsatz des "Rohproduktgemisches" aus der Gasphasenphosgenierung kann eine eintretende Verschmutzung des beschriebenen Kühlers vor Eintritt in die Quenche sein. Ursache dafür können unerwünschte Nebenprodukte bzw. Polymerverbindungen aus der Phosgenierungsreaktion sein. Auch in EP 1935875 B1 wird darauf hingewiesen, dass das Düsendesign der Kühlflüssigkeit so gewählt werden muss, dass das in die Quenche eintretende heiße Reaktionsgas nicht die relativ kalten Wände der Kühlzonen berührt und Feststoffablagerungen zu vermeiden. Neben dem Kondensationsgemisch im Sammelbehälter wird nach der Kühlstrecke ein Gasstrom, enthaltend wenigstens Chlorwasserstoff, Phosgen, ggf. Lösungsmittel, und das hergestellte Isocyanat, erhalten. Dieser Gasstrom wird dem Sammelbehälter entnommen und einer Waschkolonne zugeführt und dort weitgehend von seinen Isocyanatanteilen befreit. Bevorzugt erfolgt diese Wäsche im Gegenstrom mit Lösungsmittel. Die so erhaltene Waschphase, bestehend aus Diisocyanat und zum überwiegenden Teil aus Lösungsmittel, wird in einer bevorzugten Ausführungsform als Quench-Flüssigkeit der ersten Kühlzone der Kühlstrecke eingesetzt. Das Restgas aus der Waschkolonne besteht im Wesentlichen aus Phosgen, Chlorwasserstoff und Lösungsmittel. Diese Brüden verlassen die Kolonne über Kopf, wobei mittels Teilkondensation in einer bevorzugten Ausführungsform über zwei Kondensatoren mit unterschiedlichen Kühlmitteltemperaturen der Lösungsmittelanteil weitgehend zurückgehalten wird und auf die Kolonne als Partialkondensat zurückgeführt wird. Das danach erhaltene Restgas, das im Wesentlichen aus Phosgen, Chlorwasserstoff und Lösungsmittelresten besteht, wird anschließend in an sich bekannter Weise, wie z. B. in EP 1 849 767 B1 beschrieben, weiter behandelt.

In EP 1 935 876 A1 wird ebenfalls der Einsatz verschiedener geeigneter Quench-Flüssigkeitsströme erwähnt. Dabei wird auch auf den Einsatz der Waschflüssigkeit aus der Gaswäsche der den Kondensatsammelbehälter nach der Quenche verlassenden Brüden als Quench-Flüssigkeit hingewiesen. Die Reaktion wird hier adiabat geführt. In den erfindungsgemäßen Beispielen sind die Reaktoren entsprechend isoliert, um Wärmeverluste weitestgehend zu vermeiden, beschrieben. Im nicht isolierten Reaktor des nicht erfindungsgemäßen Beispiels entstehen im Versuch Anbackungen an den Wänden, die zum Abbruch des Versuchs durch Druckanstieg führen.

Nach der Lehre von EP-A-1 362 847 führen Temperaturschwankungen und Asymmetrien in der Temperaturverteilung zur Bildung von Nebenprodukten, die zu Anbackungen und Verstopfungen im Gasphasenreaktor führen.

Auf mehrere Kühlzonen in der Quench-Stufe weist auch EP 2 196 455 A1 hin. Auf den integrierten Verbund der Kühlzonen mehrerer Reaktoren mit einer Quench-Stufe wird hier erstmals hingewiesen. Auch in EP 2 196 455 A1 wird darauf hingewiesen, dass weder Mischräume oder Reaktionsraum Kühlflächen zulassen, die Anlass zu einer Kondensation mit der Folge von Ablagerungen geben können.

WO 2010/063665 A1 verweist auf ein mögliches Problem der bisher genannten Quenche-Varianten. Wird zumindest ein Teil der Quench-Flüssigkeit aus dem Sammelbehälter nach der Quenche, also das Diisocyanat-Rohprodukt-Lösungsmittelgemisch, entnommen, besteht die Möglichkeit, dass Feststoffe enthalten sein können, die die Quench-Düsen verstopfen können. Es werden verschiedene Techniken, wie z. B. Zentrifugieren, Abdestillieren des zur Quenche vorgesehenen Flüssigkeitsanteils, oder Filtrieren beschrieben. Um die Temperatur des ausgewählten Quenche-Stromes für die gestellte Aufgabe einzustellen, kann der Strom mittels eines Wärmetauschers gekühlt, bzw. aufgeheizt werden.

In WO 2010/115908 A2 wird eine bestimmte Ausführungsform der Quenche offenbart. Um Folgereaktionen des Reaktionsgases bei, bzw. nach der Quenchestufe zu verhindern, werden die Quenchdüsen und deren Anordnung derart konzipiert, dass eine weitgehend vollständige Benetzung der Wandung im Quenchebereich erfolgt. Damit wird das gesamte Reaktionsgemisch erfasst. Als Quenche-Flüssigkeiten werden Lösungsmittel sowie Gemische mit Isocyanat bzw. Rohgemisch der Phosgenierreaktion, ggf. nach Partikelentfernung, vorgeschlagen. In dieser Schrift wird offenbart, dass sich durch unzureichende Wandbenetzung im Quenchebereich Kondensat an kühleren Wandzonen bilden kann, das aufgrund zu langsamen Abflusses zu Folgereaktionen mit Feststoffablagerungen führen kann.

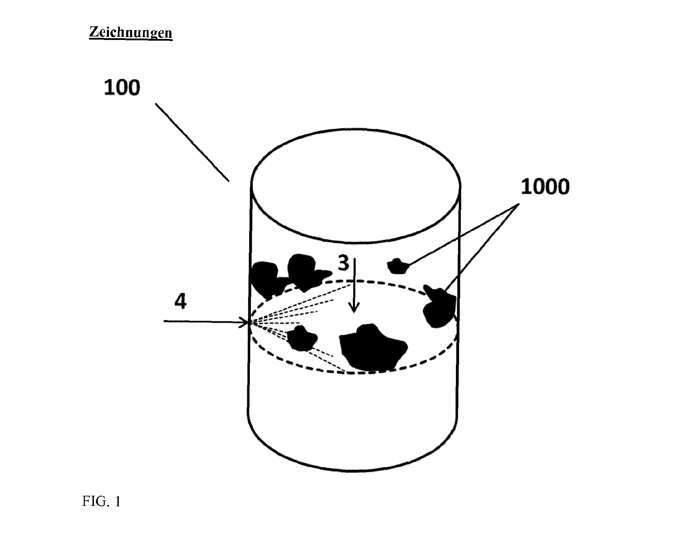

Die genannten Beschreibungen gehen nicht auf ein grundsätzliches Problem beim Betrieb des Reaktors (einschließlich der Kühlzone ("Quenche")) bei der Phosgenierung in der Gasphase ein. Es wird beim Betrieb beobachtet, dass sich an den Reaktorwänden, dort wo diese noch nicht mit Flüssigkeit benetzt sind, mit der Zeit Feststoff anlagert. Der Ursprung des Feststoffs ist nicht restlos bekannt. Ohne sich auf eine Theorie festlegen zu wollen wird die Vermutung geäußert, dass es sich um höhermolekulare Nebenprodukte aus der Reaktion handelt. Verstärkt treten diese Ablagerungen in Bereich der Quenche-Düsen auf. Dort können die Ablagerungen so stark anwachsen, dass es zu einer deutlich Verengung des effektiven freien Querschnitts des Reaktors kommt und zu einem entsprechen hohen Druckverlust. Schließlich kann sogar der Fall eintreten, dass die Produktion beendet werden muss, nämlich dann, wenn der Druckverlust zu hoch bzw. kein freier Durchlass mehr vorhanden ist. **FIG. 1** stellt diese Situation bildlich dar: Ein Produktgasstrom 3 durchläuft einen Reaktor **100** und wird von einer Quenchflüssigkeit **4** abgekühlt. Die gestrichelte Linie auf Höhe der Quenchdüse illustriert die Grenze zwischen Reaktionszone und Quenchzone: Die Reaktionszone ist oberhalb der Quenchdüse und die Quenchzone unterhalb der Quenchdüse. Oberhalb der gestrichelten Linie entlang des Umfangs des Reaktors (also vor Eintritt in die eigentliche Quenchzone) kommt es zur Bildung von Ablagerungen **1000.**

Es bestand daher ein Bedarf an einem Verfahren zur Herstellung von Diisocyanaten durch Umsetzung der entsprechenden Diamine mit Phosgen in der Gasphase, bei dem die Verschmutzung der Wand von Reaktor und Kühlzone durch Feststoffanlagerungen möglichst gering ausfällt.

Dem vorstehend Gesagten Rechnung tragend ist ein Gegenstand der vorliegenden Erfindung ein Verfahren zur Herstellung eines Isocyanats durch Umsetzung des korrespondierenden primären Amins mit Phosgen in einem Reaktor **100,** der mindestens eine Reaktionszone **110** und eine darunter angeordnete Quenchzone **120** umfasst, umfassend die Schritte:

(i) Einleiten eines gasförmigen Aminstroms **1** und eines gasförmigen Phosgenstroms **2** in den Reaktor **100** und Umsetzung in der Reaktionszone **110** zu einem Produktgasstrom **3** umfassend Isocyanat und Chlorwasserstoff sowie ggf. überschüssiges Phosgen;

(ii) Einleiten des Produktgasstromes **3** in die Quenchzone **120,** in welcher der Produktgasstrom durch Eindüsen einer Quenchflüssigkeit **4** über mindestens eine Quenchdüse **200** so abgekühlt wird, dass ein flüssiger Strom **5** umfassend Quenchflüssigkeit **4** und Isocyanat sowie ein gasförmiger Strom **6** umfassend Chlorwasserstoff und ggf. Phosgen erhalten werden;

(iii) Isolierung des Isocyanats aus dem in Schritt (ii) erhaltenen flüssigen Strom **5;**

bei dem die Temperatur der Wand der Reaktionszone **110** unmittelbar oberhalb der Quenchzone **120, $T_W^*$,** auf einem Wert gehalten wird, der maximal 4,0 %, bevorzugt maximal 2,0 %, unter der in Kelvin angegebenen Maximaltemperatur der Wand der Reaktionszone, **$T_w^{max}$,** liegt.

Das Wort *"ein"* ist im Rahmen dieser Erfindung im Zusammenhang mit zählbaren Größen nur dann als Zahlwort zu verstehen, wenn dies ausdrücklich gesagt wird (z. B. durch den Ausdruck *"genau ein"*). Bspw. schließt der Ausdruck *"ein Reaktor"* die Möglichkeit des Vorhandenseins mehrerer (in Reihe oder parallel geschalteter) Reaktoren nicht aus.

Zur näheren Erläuterung der Begriffe *"$T_W^*$"* und "*$T_w^{max}$*" wird auf die beigefügten Zeichnungen verwiesen:

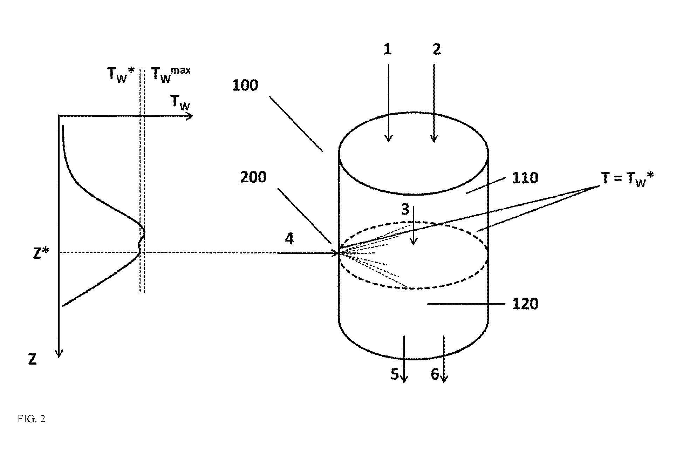

**FIG. 2** zeigt auf der rechten Seite eine schematische Darstellung eines Reaktors **100** und der einzelnen Stoffströme. Die Quenchflüssigkeit **4** wird über eine Quenchdüse **200** (nicht gezeigt) in den Reaktor eingedüst. Der Einfachheit halber ist nur ein Strom **4** gezeigt; bevorzugt gibt es deren mehrere, die um den bevorzugt zylindrischen Reaktor **100** herum angeordnet sind (nähere Details werden weiter unten erläutert). Die Lokalisation der in Strömungsrichtung des Produktgases **3** ersten Quenchdüse definiert den Übergang zwischen Reaktionszone **110** und Quenchzone **120** (in der Zeichnung durch die gestrichelte, den Reaktor umlaufende Line angedeutet). Auf der linken Seite von FIG. 2 ist das Temperaturprofil der Reaktorwand schematisch dargestellt: Infolge der Exothermie der Reaktion zwischen **1** und **2** steigt die Wandtemperatur Tw zunächst auf einen Maximalwert $T_w^{max}$ an. In der gezeigten Anordnung, in der die Quenchflüssigkeit **4** senkrecht zur Strömungsrichtung des Produktgases **3** in den Reaktor **100** gedüst wird, kommt es unter anderem infolge auch nach oben spritzender Quenchflüssigkeit zu einem Temperaturabfall bereits oberhalb der Quenche auf einen Wert **$T_W^*$.** Im Bereich unterhalb der Quenchdüse sinkt die Temperatur rapide infolge der innigen Durchmischung von Produktgasstrom **3** mit der Quenchflüssigkeit **4** sowie der Benetzung der Wand mit verhältnismäßig kalter Quenchflüssigkeit 4 und verhältnismäßig kaltem Strom 5.

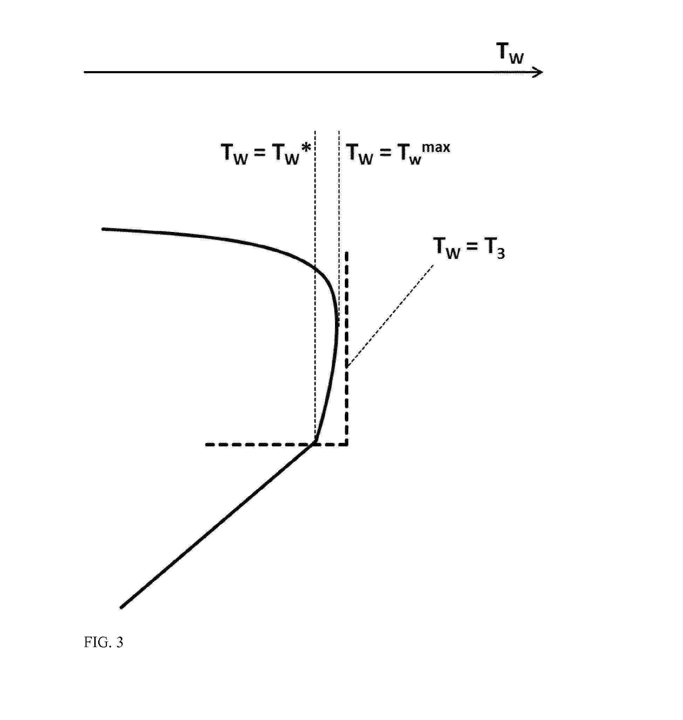

**FIG. 3** zeigt in einer Ausschnittsvergrößerung den Zusammenhang zwischen den einzelnen Temperaturen. Erfindungsgemäß wesentlich ist nun, dass **$T_W^*$** maximal 4,0 %, bevorzugt maximal 2,0 %, unter der in Kelvin angegebenen Maximaltemperatur der Wand der Reaktionszone, **$T_w^{max}$,** ist. Dies bedeutet, dass

$$100\,\% \cdot [(T_w^{max} / K) - (T_w^* / K)] / (T_w^{max} / K) \leq 4{,}0\,\% \text{ (bevorzugt} \leq 2{,}0\,\%)$$

sein muss. In FIG. 3 ist ebenfalls der theoretische optimale Temperaturverlauf gestrichelt eingezeichnet: Vor Eintritt in die Quenche ist die Temperatur der Wand des Reaktors gleich der Temperatur des Produktgases **3, $T_3$,** bei Eintritt in

die Quenche sinkt die Temperatur der Wand des Reaktors schlagartig ab.

Zur Bestimmung von $T_W^{max}$ werden mehrere Temperaturfühler (z. B. Auflagethermometer) an der Außenwand des Reaktors über die Länge und den Umfang des Reaktors verteilt. Die Anzahl der erforderlichen Temperaturfühler ist abhängig von der Größe des Reaktors. Für den Fachmann ist leicht zu ermitteln, wie viele Temperaturfühler bei gegebener Reaktorgröße und -geometrie erforderlich sind. Als $T_W^{max}$ gilt erfindungsgemäß die maximale festgestellte Temperatur. $T_W^*$ wird über einen oder mehrere Temperaturfühler auf dem Umfang direkt oberhalb der Stutzen der Quenchdüsen **200** gemessen. Bei Vorhandensein mehrerer in Strömungsrichtung des Produktgastroms **3** hintereinander angeordneter Quenchdüsen **200** wird $T_W^*$ direkt oberhalb der in Strömungsrichtung des Produktgastroms 3 ersten Quenchdüsen **200** gemessen. Auch hier ist die Anzahl der erforderlichen Temperaturfühler abhängig von der Größe des Reaktors. Für den Fachmann ist wiederum leicht zu ermitteln, wie viele Temperaturfühler bei gegebener Reaktorgröße und -geometrie erforderlich sind.

Die auf diese Weise an der Außenwand des Reaktors gemessenen Temperaturen spiegeln die Verhältnisse an der Innenwand des Reaktors, auf die es eigentlich ankommt, hinreichend gut genug wieder, denn verschiedene Faktoren führen in der Praxis zu einer vernachlässigbar kleinen Differenz zwischen den wie zuvor beschrieben gemessenen Temperaturen und denen an der Innenwand:

- Metallische, gut leitende Wandmaterialien
- Relativ geringe Dicke der Reaktorwand
- Isolation der Reaktorwand zur Umgebung hin

Nachstehend wird die Erfindung anhand verschiedener Ausführungsformen im Detail erläutert. Dabei können verschiedene Ausführungsformen beliebig miteinander kombiniert werden, sofern sich für den Fachmann aus dem Zusammenhang nicht eindeutig das Gegenteil ergibt.

Mit der vorliegenden Erfindung können solche Isocyanate, die sich unzersetzt verdampfen lassen, hergestellt werden.

Geeignete *primäre Amine* für die Durchführung des erfindungsgemäßen Verfahrens sind solche, die sich unzersetzt verdampfen lassen, insbesondere die Isomeren des Toluylendiamins (nachfolgend TDA), die Isomeren des Methylendiphenyldiamins (nachfolgend MDA), die Isomeren des Naphthyldiamins (nachfolgend NDA), 1,6-Hexamethylendiamin (nachfolgend HDA), die Isomeren des Isophorondiamins (nachfolgend IDPA) und die Isomeren des Diaminodicyclohexylmethans (nachfolgend H12-MDA). Sofern die genannten Amine in verschiedenen isomeren Formen vorliegen können, ohne dass diese angegeben sind, sind alle Isomerenverteilungen umfasst.

Besonders bevorzugt ist TDA. Üblicherweise umfasst TDA, welches bevorzugt eingesetzt wird, 78 Massen-% bis 82 Massen-% 2,4-TDA und 18 Massen-% bis 22 Massen-% 2,6-TDA, bezogen auf die Gesamtmasse der 2,4- und 2,6-TDA-Isomeren. Bezogen auf die Gesamtmasse des TDA machen dabei die 2,4- und 2,6-TDA-Isomeren bevorzugt in Summe von 95,0 Massen-% bis 100 Massen-%, besonders bevorzugt von 98,0 Massen-% bis 100 Massen-%, aus. Bevorzugt beträgt der Gehalt an TDA-Isomeren mit zueinander ortho-ständigen $NH_2$-Gruppen im einzusetzenden TDA weniger als 0,2 Massen-%, bezogen auf die Gesamtmasse des einzusetzenden TDA. Verfahren zur Bereitstellung von TDA mit den genannten Anforderungen sind dem Fachmann bekannt.

Methoden zur Bereitstellung eines gasförmigen Aminstroms 1 und eines gasförmigen Phosgenstroms **2** für die Durchführung von **Schritt (i)** sind dem Fachmann grundsätzlich bekannt. Im Folgenden werden bevorzugte Ausführungsformen geschildert.

Die Überführung des Amins in die Gasphase kann in allen aus dem Stand der Technik bekannten Verdampfungsapparaturen erfolgen. Bevorzugt werden solche Verdampfungsapparaturen eingesetzt, bei denen ein kleiner Arbeitsinhalt mit einer hohen Umwälzleistung über einen Fallfilmverdampfer geführt wird und dabei zur Minimierung der thermischen Belastung der Ausgangsamine der Verdampfungsvorgang - wie oben ausgeführt - ggf. durch Zuspeisung von Inertgas und / oder Dämpfen eines inerten Lösungsmittels unterstützt wird.

In einer bevorzugten Ausführungsform des erfindungsgemäßen Verfahrens werden Verdampfungsapparaturen eingesetzt, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Aminen wird z. B. in EP 1 754 698 A2 offenbart. Bevorzugt werden in dem erfindungsgemäßen Verfahren die in den Absätzen [0007] bis [0008] und [0017] bis [0039] der in EP 1 754 698 A2 offenbarten Apparate eingesetzt.

Weiterhin erfolgt die Verdampfung - und erforderlichenfalls Überhitzung - des Ausgangsamins bevorzugt mehrstufig, um unverdampfte Tröpfchen im gasförmigen Aminstrom 2 zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind dem Fachmann bekannt. Nach Verlassen des in Strömungsrichtung letzten Überhitzers wird das auf seine Solltemperatur vorgeheizte gasförmige Amin mit einer mittleren Verweilzeit von bevorzugt 0,01 s bis 60 s, ganz besonders bevorzugt von 0,01 s bis 30 s, insbesondere bevorzugt 0,01 s bis 15 s, der Reaktionszone **110** zur Umsetzung zugeführt. Unabhängig von der Ausgestaltung der Zuführung des Amins im Detail wird über technische Maßnahmen, z. B. eine ausreichende Isolierung zur Vermeidung von Abstrahlungsverlusten, der Gefahr einer erneuten Tröpfchenbildung begegnet.

Bei dem erfindungsgemäßen Verfahren ist es vorteilhaft, Phosgen im Überschuss bezüglich der umzusetzenden Amingruppen einzusetzen. In diesem Fall enthalten der Produktgasstrom **3** und der Gasstrom **6** auch überschüssiges Phosgen. Bevorzugt liegt ein molares Verhältnis von Phosgen zu Amingruppen von 1,1 : 1 bis 20 : 1, besonders bevorzugt 1,2 : 1 bis 5 : 1 vor. Auch das Phosgen wird auf Temperaturen von 200 °C bis 600 °C erhitzt und gegebenenfalls verdünnt mit einem Inertgas wie $N_2$, He, Ar oder mit den Dämpfen eines inerten Lösungsmittels, z. B. aromatischen Kohlenwasserstoffen ohne oder mit Halogensubstitution, wie z. B. Chlorbenzol oder o-Dichlorbenzol, der Reaktionszone **110** zugeführt (Strom **2**).

Die getrennt aufgeheizten Reaktionspartner **1** und **2** werden bevorzugt über eine Düsenanordnung in die Reaktionszone **110** des Reaktors **100** eingeführt und dort bevorzugt adiabat umgesetzt. Adiabate Umsetzung bedeutet, dass auf eine gezielte Abfuhr der entstandenen Reaktionswärme durch ein Wärmeträgermedium verzichtet wird. Daher spiegelt sich die Reaktionsenthalpie - abgesehen von unvermeidbaren Wärmeverlusten - quantitativ in der Temperaturdifferenz von Produkt- und Eduktgasstrom wider. Die Düsenanordnung zur Einführung der Eduktgasströme **1** und **2** kann auf verschiedene dem Fachmann bekannte Arten ausgestaltet sein; Beispiele finden sich z. B. in EP 2 199 277 B1, Abschnitt [0017] bis [0019], EP 1 449 826 B1, Abschnitt [0011] bis [0012], EP 1 362 847 B1, Abschnitt [0011] bis [0012], EP 1 526 129 B1, Abschnitt [0009] bis [0011] und EP 1 555 258 B1, Abschnitt [0008] bis [0011].

Der Reaktor **100** hat bevorzugt sowohl im Bereich der Reaktionszone **110** als auch im Bereich der Quenchzone **120** einen kreissymmetrischen Querschnitt. Dabei kann der ganze Reaktor zylinderförmig sein, wie in den Zeichnungen dargestellt. Es ist jedoch auch möglich, dass sich der Querschnitt verändert wie z. B. in EP 1275639 B1, Abschnitt [0009], EP 1275640 A1, Abschnitt [0014], EP 1 403 248 B1, Abschnitt [0014] bis [0015], EP 193 5876 A1, Abschnitt [0012] bis [0016] und EP 2 196 455 B1, Abschnitte [0015] bis [0026] und [0027] bis [0030] beschrieben. Weitere Details zum Bau geeigneter Phosgenierungsreaktoren sind dem Fachmann bekannt.

In der Reaktionszone **110** werden Amin **1** und Phosgen **2** rasch zum korrespondierenden Isocyanat umgesetzt, bevorzugt adiabat. Die Reaktion wird bevorzugt so geführt, dass das Amin vor Eintritt in die Quenchzone vollständig umgesetzt ist.

In einer bevorzugten Ausführungsform des erfindungsgemäßen Verfahrens beträgt die Durchsatzkapazität des eingesetzten Reaktors **100** bei den erfindungsgemäß geforderten Reaktionsbedingungen > 1 t Amin / h, bevorzugt 2 - 50 t Amin / h, besonders bevorzugt 2 - 12 t Amin / h. Besonders bevorzugt gelten diese Werte für Toluylendiamin, 1,6-Diamino-hexan und für Isophorondiamin. Unter Durchsatzkapazität ist dabei zu verstehen, dass in dem Reaktor **100** die genannte Durchsatzkapazität an Amin pro Stunde umgesetzt werden kann.

Nach der erfolgten Phosgenierungsreaktion in der Reaktionszone **110** wird das gasförmige Reaktionsgemisch **3,** das wenigstens das angestrebte Isocyanat und Chlorwasserstoff umfasst (sowie, bei überstöchiometrischem Einsatz von Phosgen auch nicht umgesetztes Phosgen), in die Quenchzone **120** geleitet **(Schritt (ii)),** wo das gebildete Isocyanat weitgehend durch Eindüsen der Quenchflüssigkeit **4** kondensiert wird. Möglichkeiten für den Aufbau und den Betrieb der Quenchzone **120** sind im Prinzip aus dem Stand der Technik bekannt. Sofern die erfindungsgemäßen Anforderungen im Hinblick auf die Temperatur $T_W^*$ eingehalten werden, können die Apparate und Methoden des Standes der Technik eingesetzt werden. Mögliche Ausgestaltungen der Quenchzone **120** sind beispielsweise in EP 1 403 248 A1 und EP 1 935 875 A1 offenbart.

Die Temperatur der Quenchflüssigkeit **4** wird bevorzugt so gewählt, dass sie einerseits hoch genug ist, um das dem Isocyanat entsprechende Carbamoylchlorid in Isocyanat und Chlorwasserstoff zurückzuspalten. (Es ist zwar keineswegs sicher, ob das aus der Flüssigphasenphosgenierung bekannte Zwischenprodukt Carbamoylclorid auch in der Gashasenphosgenierung gebildet wird. Da es jedoch unabhängig davon denkbar ist, dass in der Quenche *verflüssigtes* Isocyanat 5 mit dem vorhandenem Chlorwasserstoffgas **6** teilweise zum Carbamoylchlorid reagiert, sollte die Temperatur der Quenchflüssigkeit **4** hoch genug sein, um diese Reaktion zurückzudrängen.) Andererseits sollen Isocyanat und gegebenenfalls das in dem gasförmigen Aminstrom und / oder gasförmigen Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und ggf. als Verdünnungsmittel mit verwendetes Inertgas die Quenchzone **120** weitestgehend nicht kondensiert bzw. nicht gelöst durchlaufen, sodass die Temperatur der Quenchflüssigkeit **4** auch nicht zu hoch gewählt werden darf. Zur selektiven Gewinnung des Isocyanats aus dem gasförmigen Reaktionsgemisch **3** besonders gut geeignet sind bei einer Temperatur von 80 °C bis 200 °C, vorzugsweise 80 C bis 180 °C gehaltene Quenchflüssigkeiten **4.** Die Quenchflüssigkeit **4** ist dabei bevorzugt ausgewählt aus Chlorbenzol, Dichlorbenzol, Isocyanat und Mischungen der vorgenannten Flüssigkeiten. Es ist für den Fachmann aufgrund der physikalischen Daten bei gegebener Temperatur, Druck und Zusammensetzung einfach vorhersagbar, welcher Massenanteil des Isocyanats in der Quenche kondensiert bzw. diese nicht kondensiert durchläuft. Ebenso ist es einfach vorherzusagen, welcher Massenanteil des überschüssigen Phosgens, Chlorwasserstoffs und ggf. als Verdünnungsmittel verwendeten Inertgases die Quenche unkondensiert durchläuft bzw. sich in der Quenchflüssigkeit löst. Bevorzugt strömen die Edukt- und Produktgase ohne wesentliche Rückvermischung durch den Reaktor **100.** Dies wird durch ein Druckgefälle über die Reaktionszone **110,** bevorzugt über die Reaktionszone **110** und die sich anschließende Quenchzone **120,** sichergestellt. Bevorzugt besteht das Druckgefälle zwischen dem Beginn der Reaktionszone **110** einerseits und dem Ausgang aus der Quenchzone **120** andererseits. Bevorzugt liegt der absolute Druck am Beginn der Reaktionszone **110** bei 200 mbar bis 3000 mbar und hinter der Quenchzone **120** bei 150 mbar bis 2500 mbar. Wesentlich ist hierbei jedoch lediglich die Aufrechterhaltung eines Differenzdrucks über die Reaktionszone **110,** bevorzugt über die Reaktionszone **110** und die Quenchzone **120** von bevorzugt mindestens 50 mbar zwecks Gewährleistung der genannten gerichteten Strömung und einer guten Vermischung der Edukte.

In **Schritt (iii)** wird aus dem flüssigen Gemisch **5,** das aus der Quenchzone **120** austritt, das Isocyanat gewonnen. Dies geschieht bevorzugt durch destillative Aufarbeitung, besonders bevorzugt wie in EP 1 371 633 A1 beschrieben.

Das die Quenchzone **120** verlassende Gasgemisch **6** wird bevorzugt in einer nachgeschalteten Gaswäsche mit einer geeigneten Waschflüssigkeit von ggf. vorhandenem restlichem Isocyanat befreit und bevorzugt anschließend in an sich bekannter Weise von überschüssigem Phosgen befreit. Dies kann mittels einer Kühlfalle, Absorption in einem inerten Lösungsmittel (z. B. Chlorbenzol oder Dichlorbenzol) oder durch Adsorption und Hydrolyse an Aktivkohle erfolgen. Bevorzugt erfolgt dieser Schritt wie in WO2011/00353 A1 beschrieben; siehe insbesondere S. 11, Z. 31 bis S. 25, Z. 15. Bevorzugt wird das aus dem Gasgemisch abgetrennte überschüssige Phosgen wieder dem Reaktor zugeführt. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die nach ihrem Einsatz zur Gaswäsche anfallende Waschflüssigkeit wird dann bevorzugt zumindest teilweise als Quench-Flüssigkeit zur Abkühlung des Gasgemisches in die entsprechende Zone des Reaktionsraumes eingesetzt.

Erfindungswesentlich ist nun, dass $T_W^*$ maximal 4,0 %, bevorzugt maximal 2,0 %, unter der in Kelvin angegebenen Maximaltemperatur der Innenwand der Reaktionszone, $T_W^{max}$, ist. Es wurde nämlich gefunden, dass Ablagerungen **1000** (FIG. 1) umso weniger auftreten, je höher die Temperatur der Reaktorwand im Bereich oberhalb der Quenchzone **120** ist.

Dies kann auf verschiedene Weisen sichergestellt werden, die auch miteinander kombiniert werden können:

In einer bevorzugten Ausführungsform werden die erfindungsgemäßen Anforderungen an die Temperatur $T_W^*$ durch mindestens eine der folgenden Maßnahmen sichergestellt:

(a) Isolation der Reaktorwand nach außen,

(b) Beheizung der Reaktorwand von außen,

(c) Isolation zwischen Wand der Reaktionszone und Wand der Quenchzone.

Materialien und Methoden zur Isolation sind dem Fachmann bekannt. Mit Maßnahme (a) werden Wärmeverluste nach außen hin minimiert. Bei Maßnahme (b) wird die Reaktorwand oberhalb der Quenchzone **120** von außen beheizt, bevorzugt elektrisch. Hierdurch werden die Wärmeverluste durch Wärmeleitung in der Wand ausgeglichen. Maßnahme (c) unterdrückt dabei die Wärmeleitung in der Reaktorwand, verhindert also, dass sich die kälteren Wandtemperaturen in der Quenchzone **120** bis in die Reaktionszone **110** hinein auswirken.

In einer weiteren bevorzugten Ausführungsform werden die erfindungsgemäßen Anforderungen an die Temperatur $T_W^*$ durch mindestens eine der folgenden Maßnahmen sichergestellt:

(a) Erhöhung der Temperatur der Quenchflüssigkeit,

(b) Verringerung der Menge an eingedüster Quenchflüssigkeit.

Diese Maßnahmen können natürlich nicht in beliebigem Ausmaß eingesetzt werden, denn die Funktion der Quenche, die rasche Abkühlung des Produktgasstroms 3, muss gewährt bleiben. Der Fachmann kann aus den gegebenen Randbedingungen einer Produktionsanlage leicht ermitteln, welcher Mindeststrom an Quenchflüssigkeit (bei gegebener Temperatur der Quenchflüssigkeit) erforderlich ist bzw. welche Temperatur die Quenchflüssigkeit (bei gegebenem Massenstrom) maximal haben darf.

Aufbau und Anordnung der Quenchdüsen erfolgt bevorzugt so, wie in EP 1403 248 B1 offenbart.

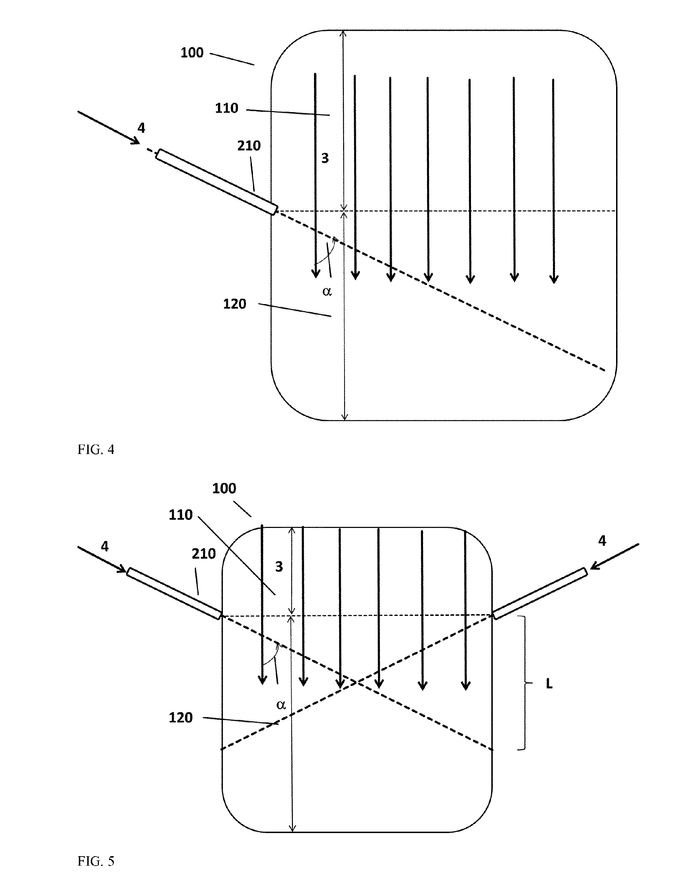

In einer weiteren bevorzugten Ausführungsform sind die Quenchdüsen **200,** bei Vorhandensein mehrerer in Strömungsrichtung des Produktgastroms 3 hintereinander angeordneter Quenchdüsen mindestens die in Strömungsrichtung des Produktgastroms 3 ersten Quenchdüsen, so ausgerichtet, dass die lineare(n) Verlängerung(en) des Düsenrohrs / der Düsenrohre **210** einen Winkel mit der Strömungsrichtung des Produktgastroms 3 von 20° bis 80°, bevorzugt von 20° bis 50°, aufweist/aufweisen (FIG. 4, Winkel α). Die *lineare Verlängerung des Düsenrohrs* entspricht dabei der Strömungsrichtung, welche die Quenchflüssigkeit hätte, wenn sie ohne jede Auffächerung als gerader Strahl aus dem Austrittsrohr der Düse (dem Düsenrohr) austreten würde. Hierdurch wird verhindert, dass Quenchflüssigkeit **4** nach oben in den Bereich der Reaktionszone **110** gesprüht wird. Ist die Quenchdüse **200** nämlich rechtwinklig zur Produktgasströmung **3** angeordnet, gelangt immer auch ein Teil der Quenchflüssigkeit **4** an die Innenwand der Reaktionszone **110** unmittelbar oberhalb der Düsen und bewirkt dort eine unerwünschte Temperaturabnahme, welche Anlass zur Bildung von Ablagerungen geben kann. Dies kann durch ein entsprechendes Design der Quenche effektiv vermindert werden. Entscheidend dafür ist es, die Sprührichtung der Quenchdüse **200** in Richtung der Gasströmung **3** auszulenken, also entsprechend nach unten zu sprühen (vgl. FIG. 4). Die Quenchflüssigkeit **4** wird dabei mit üblichen Sprühdüsen oder durch Öffnungen, beispielsweise Schlitze oder Löcher, bevorzugt als Flachstrahl eingedüst, also ohne Auffächerung in vertikaler Richtung. Es zeigte sich in Betriebsversuchen in einer Pilotanlage, dass durch einen Sprühwinkel von 20° bis 80°, bevorzugt 20° bis 50°, relativ zur Gasströmung **3** ein "Nach-oben-Sprühen" und Feststoffanlagerungen wirkungsvoll vermindert werden können. Damit werden Laufzeit und Verfügbarkeit Gasphasenreaktors **100** erhöht.

Alternativ zur Beschreibung des Eindüswinkels kann der Einfluss der Düsengeometrie auch über den Abstand L beschrieben werden, der sich für eine bestimmte Quencheanordnung zwischen der Quencheebene und der Ebene ergibt, auf der die Quencheflüssigkeit auf die Reaktorwand trifft. Je nach Auffächerung der Düse ist dieser Abstand nicht einheitlich, so dass folgende vereinfachte Darstellung gewählt wird: Als Abstand L wird der Abstand zwischen der Quencheebene und dem gedachten Schnittpunkt der Verlängerung der Eindüsrichtung und der Reaktorwand gemäß Abbildung 5 definiert. Der Begriff Quencheebene meint dabei die gedachte Ebene durch den Punkt, an dem die Austrittsöffnung der in Strömungsrichtung des Produktgases ersten Quenchdüse in das Innere des Reaktors ragt; dies wird erfindungsgemäß als Beginn der Quenchzone aufgefasst. *Für eine gegebene Reaktorgeometrie* lassen sich die beiden Maße Eindüswinkel $\alpha$ und Abstand zur Quenchebene L durch den Fachmann leicht ineinander überführen. Entscheidender Unterschied zwischen den beiden Maßen ist, dass sich der Abstand zur Quenchebene L mit verändertem Reaktordurchmesser nicht verändert. Es wurde nun gefunden, dass es für den Wert L einen bevorzugten Mindestwert gibt, der - unabhängig vom Wert des Eindüswinkels $\alpha$ - bevorzugt nicht unterschritten werden sollte. In einer weiteren Ausführungsform des erfindungsgemäßen Verfahrens sind daher die Quenchdüsen **200,** bei Vorhandensein mehrerer in Strömungsrichtung des Produktgastroms **3** hintereinander angeordneter Quenchdüsen mindestens die in Strömungsrichtung des Produktgastroms **3** ersten Quenchdüsen, so angeordnet, dass die lineare Verlängerung des Düsenrohrs die Wand der Quenchzone an einer Stelle kreuzt, die mindestens um den Abstand L unterhalb des Beginns der Quenchzone liegt (vgl. FIG. 5). Der Wert L beträgt erfindungsgemäß 15 cm, bevorzugt 70 cm.

## Beispiele

### Beispiel 1: Abhängigkeit des Temperaturprofils der Reaktorwand vom Eindüswinkel $\alpha$ bzw. vom Abstand L

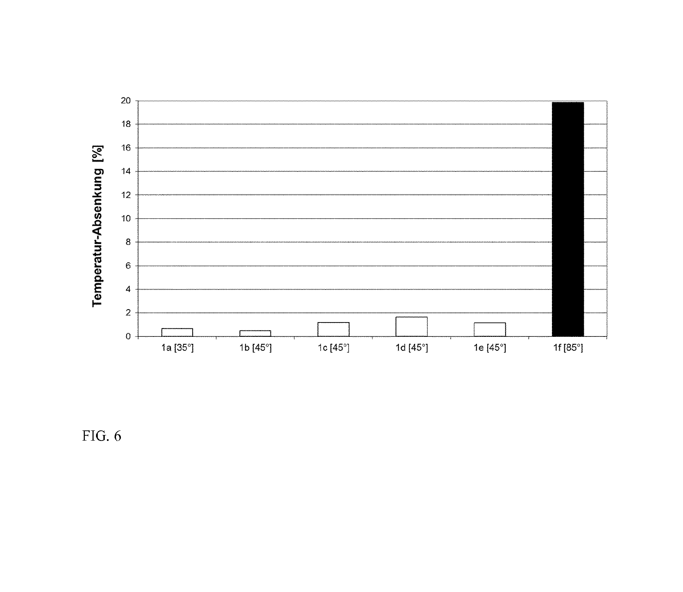

Abbildung 6 (FIG. 6) zeigt Versuchsergebnisse einer Studie zum Einfluss des Eindüswinkels $\alpha$ auf die Absenkung der Wandtemperatur im Bereich der Quenche. Die Studie wurde in einer Versuchseinrichtung im Pilotmaßstab zur Produktion von Toluylendiisocyanat mit Hilfe der Phosgenierung des zugehörigen Amins in der Gasphase durchgeführt. $T_W^{max}$ betrug in allen Versuchen zwischen 400 und 500 °C. Die Temperatur der Quenche-Flüssigkeit betrug 125 °C. Der Reaktor war zylindrisch mit einem konstanten Innendurchmesser von 800 mm in Reaktions- und Quenchezone. Die prozentuale Absenkung der Wandtemperatur ist dabei wie zuvor definiert als

$$100\ \%\ \cdot\ [(\mathbf{T_W^{max}}\ /\ \mathbf{K}) - (\mathbf{T_W^*}\ /\ \mathbf{K})]\ /\ (\mathbf{T_W^{max}}\ /\ \mathbf{K}).$$

| Bsp: | 1a | 1b | 1c | 1d | 1e | 1f (Vergleich) |
|---|---|---|---|---|---|---|
| $\alpha$ | 35° | 45° | 45° | 45° | 45° | 85° |

Deutlich zu erkennen ist der starke Anstieg der Temperaturabsenkung bei einer Eindüsrichtung fast senkrecht zur Strömungsrichtung des Reaktionsgemischs (85°, Balken rechts). Die Versuche mit Eindüsrichtungen von 35° und 45° (fünf Balken links) führen hingegen zu nur geringen Temperaturabsenkungen.

Abbildung 6 zeigt außerdem den Zusammenhang zwischen Temperaturabsenkung und verstärkter Feststoffablagerung. Schwarze Balken zeigen Versuchsläufe mit einer deutlich stärkeren Feststoffanlagerung an der Reaktorinnenwand im Bereich der Quenchdüsen an im Vergleich zu den anderen Versuchsläufen. Die Gefahr einer Feststoffanlagerung kann also durch die Begrenzung der Temperaturabsenkung, hier realisiert durch die geeignete Wahl des Eindüswinkels, deutlich beeinflusst werden.

Die in Abbildung 6 angegebenen Winkel lassen sich wie folgt als Abstand zwischen Aufprallebene der Quencheflüssigkeit und Quencheebene beschreiben:

| Bsp. Nr. | Eindüswinkel $\alpha$ | Abstand L |
|----------|----------------------|-----------|
| 1a | 35° | 114,3 cm |
| 1b-e | 45° | 80,0 cm |
| 1f | 85° | 7,0 cm |

Die Temperaturabsenkung konnte also durch Einstellen eines Abstands L von 80 cm gegenüber 7 cm deutlich verringert werden.

**Patentansprüche**

1. Verfahren zur Herstellung eines Isocyanats durch Umsetzung des korrespondierenden primären Amins mit Phosgen in einem Reaktor **100,** der mindestens eine Reaktionszone **110** und eine darunter angeordnete Quenchzone **120** umfasst, umfassend die Schritte:

   (i) Einleiten eines gasförmigen Aminstroms **1** und eines gasförmigen Phosgenstroms **2** in den Reaktor **100** und Umsetzung in der Reaktionszone **110** zu einem Produktgasstrom **3** umfassend Isocyanat und Chlorwasserstoff;
   (ii) Einleiten des Produktgasstromes **3** in die Quenchzone **120,** in welcher der Produktgasstrom durch Eindüsen einer Quenchflüssigkeit **4** über mindestens eine Quenchdüse **200** so abgekühlt wird, dass ein flüssiger Strom **5** umfassend Quenchflüssigkeit **4** und Isocyanat erhalten wird;
   (iii) Isolierung des Isocyanats aus dem in Schritt (ii) erhaltenen flüssigen Strom 5;
   **dadurch gekennzeichnet, dass**
   die Temperatur der Wand der Reaktionszone unmittelbar oberhalb der Quenchzone, $T_W^*$, auf einem Wert gehalten wird, der maximal 15 % unter der in Kelvin angegebenen Maximaltemperatur der Wand der Reaktionszone, $T_W^{max}$, liegt.

2. Verfahren nach Anspruch 1, bei dem die Temperatur $T_W^*$ auf einem Wert, der maximal 2,0 % unter der in Kelvin angegebenen Temperatur $T_W^{max}$, liegt, gehalten wird.

3. Verfahren nach einem der Ansprüche 1 oder 2, bei dem die Anforderung an die Temperatur $T_W^*$ durch mindestens eine der folgenden Maßnahmen erreicht wird:

   (a) Isolation der Reaktorwand nach außen,
   (b) Beheizung der Reaktorwand von außen,
   (c) Isolation zwischen Wand der Reaktionszone und Wand der Quenchzone.

4. Verfahren nach einem der Ansprüche 1 bis 3, bei dem die Anforderung an die Temperatur $T_W^*$ durch mindestens eine der folgenden Maßnahmen erreicht wird:

   (a) Erhöhung der Temperatur der Quenchflüssigkeit,
   (b) Verringerung der Menge an eingedüster Quenchflüssigkeit.

5. Verfahren nach einem der Ansprüche 1 bis 4, bei dem die Quenchdüse, bei Vorhandensein mehrerer in Strömungsrichtung des Produktgastroms **3** hintereinander angeordneter Quenchdüsen mindestens die in Strömungsrichtung des Produktgastroms **3** ersten Quenchdüsen, so ausgerichtet sind, dass die lineare Verlängerung des Düsenrohrs / der Düsenrohre einen Winkel mit der Strömungsrichtung des Produktgastroms **3** von 20° bis 80° aufweist/aufweisen.

6. Verfahren nach einem der Ansprüche 1 bis 4, bei dem die Quenchdüse, bei Vorhandensein mehrerer in Strömungsrichtung des Produktgastroms **3** hintereinander angeordneter Quenchdüsen mindestens die in Strömungsrichtung des Produktgastroms **3** ersten Quenchdüsen, so ausgerichtet sind, dass die lineare Verlängerung des Düsenrohrs / der Düsenrohre einen Winkel mit der Strömungsrichtung des Produktgastroms **3** von 50° bis 80° aufweist/aufweisen.

7. Verfahren nach einem der Ansprüche 1 bis 5, bei dem die Quenchdüse, bei Vorhandensein mehrerer in Strömungsrichtung des Produktgastroms **3** hintereinander angeordneter Quenchdüsen mindestens die in Strömungsrichtung des Produktgastroms **3** ersten Quenchdüsen, so ausgerichtet ist/sind, dass die lineare Verlängerung des Düsenrohrs die Wand der Quenchzone an einer Stelle kreuzt, die mindestens um den Abstand L unterhalb des Beginns der Quenchzone liegt, wobei L = 15 cm ist.

8. Verfahren nach einem der Ansprüche 1 bis 5, bei dem die Quenchdüse, bei Vorhandensein mehrerer in Strömungsrichtung des Produktgastroms **3** hintereinander angeordneter Quenchdüsen mindestens die in Strömungsrichtung des Produktgastroms **3** ersten Quenchdüsen, so ausgerichtet ist/sind, dass die lineare Verlängerung des Düsenrohrs die Wand der Quenchzone an einer Stelle kreuzt, die mindestens um den Abstand L unterhalb des Beginns der Quenchzone liegt, wobei L = 70 cm ist.

9. Verfahren nach einem der Ansprüche 1 bis 8, bei dem das primäre Amin ausgewählt ist aus der Gruppe bestehend aus den Isomeren des Toluylendiamins, den Isomeren des Methylendiphenyldiamins, den Isomeren des Naphthyldiamins, 1,6-Hexamethylendiamin, den Isomeren des Isophorendiamins und den Isomeren des Diaminodicyclohexylmethans.

Zeichnungen

100

1000

3

4

1000

FIG. 1

FIG. 2

FIG. 3

FIG. 4

FIG. 5

FIG. 6

Europäisches
Patentamt

European
Patent Office

Office européen
des brevets

# EUROPÄISCHER RECHERCHENBERICHT

Nummer der Anmeldung

EP 13 17 8270

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 1 403 248 A1 (BAYER AG [DE] BAYER MATERIALSCIENCE AG [DE]) 31. März 2004 (2004-03-31) * das ganze Dokument * ----- | 1-9 | INV. C07C263/10 |
| A,D | EP 1 935 875 A1 (BAYER MATERIALSCIENCE AG [DE]) 25. Juni 2008 (2008-06-25) * das ganze Dokument * ----- | 1-9 | |
| A,D | WO 2010/115908 A2 (BASF SE [DE]; LEHR VANESSA SIMONE [DE]; KNOESCHE CARSTEN [DE]; MATTKE) 14. Oktober 2010 (2010-10-14) * das ganze Dokument * ----- | 1-9 | |
| A | WO 2011/113737 A1 (BASF SE [DE]; LEHR VANESSA SIMONE [DE]; MATTKE TORSTEN [DE]) 22. September 2011 (2011-09-22) * das ganze Dokument * ----- | 1-9 | |
| A | WO 2010/100221 A1 (BASF SE [DE]; KNOESCHE CARSTEN [DE]; MATTKE TORSTEN [DE]) 10. September 2010 (2010-09-10) * das ganze Dokument * ----- | 1-9 | RECHERCHIERTE SACHGEBIETE (IPC) C07C |

Der vorliegende Recherchenbericht wurde für alle Patentansprüche erstellt

| Recherchenort | Abschlußdatum der Recherche | Prüfer |
|---|---|---|
| Den Haag | 2. April 2014 | Megido, Benigno |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 13 17 8270

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-04-2014

| Im Recherchenbericht angeführtes Patentdokument | Datum der Veröffentlichung | Mitglied(er) der Patentfamilie | Datum der Veröffentlichung |
|---|---|---|---|
| EP 1403248 A1 | 31-03-2004 | AT 327217 T | 15-06-2006 |
| | | CA 2442467 A1 | 30-03-2004 |
| | | CN 1496977 A | 19-05-2004 |
| | | DE 10245704 A1 | 01-04-2004 |
| | | EP 1403248 A1 | 31-03-2004 |
| | | ES 2265543 T3 | 16-02-2007 |
| | | HK 1065998 A1 | 25-01-2008 |
| | | JP 4486800 B2 | 23-06-2010 |
| | | JP 2004123745 A | 22-04-2004 |
| | | MX PA03008808 A | 15-04-2004 |
| | | US 2004068137 A1 | 08-04-2004 |
| EP 1935875 A1 | 25-06-2008 | AT 517082 T | 15-08-2011 |
| | | CN 101200437 A | 18-06-2008 |
| | | DE 102006058634 A1 | 19-06-2008 |
| | | EP 1935875 A1 | 25-06-2008 |
| | | JP 5366393 B2 | 11-12-2013 |
| | | JP 2008150372 A | 03-07-2008 |
| | | KR 20080055661 A | 19-06-2008 |
| | | TW 200842122 A | 01-11-2008 |
| | | US 2008167490 A1 | 10-07-2008 |
| WO 2010115908 A2 | 14-10-2010 | CN 102369182 A | 07-03-2012 |
| | | EP 2417100 A2 | 15-02-2012 |
| | | JP 2012523389 A | 04-10-2012 |
| | | KR 20120013937 A | 15-02-2012 |
| | | US 2012004445 A1 | 05-01-2012 |
| | | WO 2010115908 A2 | 14-10-2010 |
| WO 2011113737 A1 | 22-09-2011 | CN 102803206 A | 28-11-2012 |
| | | EP 2547652 A1 | 23-01-2013 |
| | | JP 2013522261 A | 13-06-2013 |
| | | KR 20130029064 A | 21-03-2013 |
| | | WO 2011113737 A1 | 22-09-2011 |
| WO 2010100221 A1 | 10-09-2010 | CN 102341369 A | 01-02-2012 |
| | | EP 2403826 A1 | 11-01-2012 |
| | | JP 2012519667 A | 30-08-2012 |
| | | KR 20110135937 A | 20-12-2011 |
| | | US 2011301380 A1 | 08-12-2011 |
| | | WO 2010100221 A1 | 10-09-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0289840 B1
- EP 0749958 B1
- DE 10158160 A1
- EP 1761483 B1
- WO 2007014936 A2
- WO 2011003532 A1
- EP 1403248 B1
- EP 1935875 B1
- EP 1849767 B1
- EP 1935876 A1
- EP 1362847 A
- EP 2196455 A1
- WO 2010063665 A1
- WO 2010115908 A2
- EP 1754698 A2
- EP 2199277 B1
- EP 1449826 B1
- EP 1362847 B1
- EP 1526129 B1
- EP 1555258 B1
- EP 1275639 B1
- EP 1275640 A1
- EP 2196455 B1
- EP 1403248 A1
- EP 1935875 A1
- EP 1371633 A1
- WO 201100353 A1